Europäisches Patentamt

European Patent Office

Office européen des brevets

(19)

(11) Publication number: **0 424 915 A2**

## (12) EUROPEAN PATENT APPLICATION

(21) Application number: **90120417.2**

(22) Date of filing: **24.10.90**

(51) Int. Cl.⁵: **C12P 31/00, C07C 405/00**

(30) Priority: **25.10.89 JP 277560/89**

(43) Date of publication of application:
**02.05.91 Bulletin 91/18**

(84) Designated Contracting States:
**AT BE CH DE ES FR GB IT LI NL SE**

(71) Applicant: **LION CORPORATION**
**3-7, Honjo 1-chome**
**Sumida-ku Tokyo(JP)**

(72) Inventor: **Nakajima, Ichiro**
**5515, Tamura**
**Hiratsuka-shi, Kanagawa-ken(JP)**
Inventor: **Suzaki, Kazuhiko**
**3-17-34, Fujimigaoka, Ninomiya-machi**
**Naka-gun, Kanagawa-ken(JP)**
Inventor: **Tsuji, Masahisa**
**457, Yamanishi Ninomiya-machi**
**Naka-gun, Kanagawa-ken(JP)**

(74) Representative: **Vossius & Partner**
**Siebertstrasse 4 P.O. Box 86 07 67**
**W-8000 München 86(DE)**

(54) Methods for producing prostaglandins and for purifying the same.

(57) A method for producing prostaglandins comprises converting unsaturated fatty acids contained in an alga belonging to genus Gracilaria or genus Hypnea into prostaglandins by the action of an enzyme present in the alga in water or a water-containing solvent so that the prostaglandins are released from the alga into the water or the water-containing solvent and then recovering the prostaglandins from the solvent; or comprises adding an unsaturated fatty acid to water or a water-containing solvent, converting the unsaturated fatty acid into prostaglandins by the action of the enzyme present in the alga belonging to genus Gracilaria or genus Hypnea in the solvent and then recovering prostaglandins from the solvent. The purification of prostaglandins can effectively be performed by a method which comprises contacting a solution of prostaglandins in water or a water-containing organic solvent with a non-polar porous synthetic resin having a specific surface area of not less than 350 m²/g and a fine pore volume of not less than 0.8 mℓ /g to thus adsorb the prostaglandins to the resin and then eluting the adsorbed prostaglandins with an organic solvent or a water-containing organic solvent for elution. The method makes it possible to provide desired prostaglandins in high yield and to produce prostaglandins in industrial scale.

EP 0 424 915 A2

# METHODS FOR PRODUCING PROSTAGLANDINS AND FOR PURIFYING THE SAME

The present invention relates to a biosynthetic method for producing prostaglandins by converting unsaturated fatty acids contained in alga with an enzyme present in the specific algae as well as a method for purifying prostaglandins in which a specific non-polar porous synthetic resin is employed.

The clue to the discovery of prostaglandins was the discovery of a substance present in the human semen which can serve to contract uteri and was discovered by Kurzrok and Lieb who are gynecologists of Colombia University of the United States and have conducted studies of artificial fertilization for married couples who are not blessed with children, and thereafter many research workers have conducted intensive studies of such a substance and the chemical structures of the prostaglandins are already revealed through these studies. In addition, the studies thereof have widely been conducted to elucidate the physiological activity and the pharmacological action thereof.

With regard to the application of prostaglandins as medicines, Prostaglandin $E_1$ (hereinafter referred to as "$PGE_1$") is approved as a medicine for treating chronic arterial obstruction and prostaglandin $E_2$ - (hereinafter referred to as "$PGE_2$") and Prostaglandin $F_2 \alpha$ (hereinafter referred to as "$PGF_2\alpha$") are also approved as aparturifaciens in the field of obsterics and gynecology. The $PGF_2 \alpha$ is also approved as a promotor of intestinal movement after abdominal operations. In response to the application of prostaglandins as medicines, industrial methods for producing prostaglandins per se have been investigated from various angles.

There have been proposed a variety of characteristic chemical synthetic methods. Inter alia, typical examples thereof are a method of Corey et al. (E.J. Corey, N.M. Weinshenker, T.K. Schaaf, W. Huber, J. Am. Chem. Soc., 1969, 91 , p. 5675) in which a prostaglandin is prepared from cyclopentadiene through an intermediate called Corey lactone; and a three-component method of Noyori et al. (M. Suzuki, A. Yanagisawa, R. Noyori, J. Am. Chem. Soc., 1985, 107, p. 3348) which comprises performing 1,4-addition reaction of a cyclopentenone derivative to introduce an $\omega$ -chain while trapping the resulting enolate through an $\alpha$ -chain to thus synthesize a prostaglandin at one step. However, these chemical synthetic methods suffer from a problem in that they need many synthetic processes and the yield of the prostaglandin is very low.

In addition to overall chemical synthetic methods, there have been proposed a semi-synthetic method for producing prostaglandin in which an extract from the coral is used as a starting material (see E. J. Corey, H.E. Ensley, J. Org. Chem., 1973,38, p. 3187). In 1969, it was found out that a very large amount of prostaglandin $A_2$ (hereinafter referred to as "$PGA_2$") and isomers thereof are included in the coral which is the coelenteron living in the Caribbean Sea and thus the path for the synthesis of $PGE_2$ through the $PGA_2$ as an intermediate has been developed. However, this method suffers from various problems. For instance, this method requires great expense for extracting the unstable substances from the coral and for purifying the extract and the method exerts adverse influence on the ecosystem of the coral (it seems to take 7 years for the regeneration of the coral after the collection thereof).

Moreover, there have been proposed biosynthetic methods developed on the basis of or in the light of biological reactions. For instance, 8 to 12 g of a prostaglandin is produced from 75 kg of a homogenate of the seminal cyst of sheep and 37 g of arachidonic acid according to biosynthetic method (in Up John Company; see E.G. Daniels, J.E. Pike, PG Symposium of the Worcester Foundation for Experimental Boilogy, 1968, p. 379, Interscience Publisher). The application of these biosynthetic methods is rather limited since they require the use of internal organs of animals. In addition, attempts have been made to eliminate drawbacks such as low stability of enzymes used and their expensiveness, but these methods are at the present state far from the stage of practical application.

In addition to these synthetic methods, there have been proposed methods for extracting prostaglandins from algae. R.P. Gregson et al. (Roch Chemical Company) report that $PGE_2$ and $PGF_2 \alpha$ can be obtained from an alga: Gracilari a lichenoide s (see R.P. Gregson, J.F. Marwood, R.J. Quinn, Tetrahedron Letters, 1979, 46 , p. 4505). Moreover, Tomita et al. report a method for preparing $PGE_2$ which comprises extracting it from an alga: Gracilaria verrucosa (Hudson) Papenfuss (see Hiroshi TOMITA, Yasumasa IKEJO, Japanese PATENT Publication for Opposition Purpose (hereunder referred to as "J.P. KOKOKU") No. Hei 1- 14910, TERUMO CORPORATION). In addition, Fusetani et al. report that $PGA_2$ and $PGE_2$ may be obtained from an alga: Gracilaria verrucosa (see N. Fusetani, K. Hashimoto, Bulletin of the Japanese Society of Scientific Fisheries 1984,50(3) , p. 465). In these methods for extracting prostaglandins from algae, the highest yield of $PGE_2$ is 96 $\mu$ g per gram of the weight of wet alga which has been achieved by the method of Tomita et al. and it has been desired for the development of an extraction method which can provide a higher yield. On the other hand, not only $PGE_2$ and $PGF_2 \alpha$ but also $PGE_1$ are presently used as medicines and the

2

demand for these medicines has markedly increased year by year. There has not been reported any method for extracting $PGE_1$ and prostaglandin $E_3$ (hereunder referred to as "$PGE_3$") from algae.

On the other hand, the prostaglandins thus produced should be highly purified by a proper treatment. The purity of the prostaglandins are important from the immunological viewpoint, stability of the prostaglandins or the like. This is because, the prostaglandins are used as a variety of medicines as has been explained above and they are very unstable and may possibly be decomposed by impurities present therein during production and/or storing.

As methods for purifying the prostaglandins, there have been known those in which organic solvents or resins are employed. This is detailed in, for instance, "Biochemistry of Prostaglandin", edited by S. MUROTA, p. 151, Tokyo Kagakudojin Publishing Company, 1982; and "GENDAI KAGAKU, extra edition No. 6, Methods for Studying Prostaglandins (the first volume)", 1986, p. 62, Tokyo Kagakudojin Publishing Company. In general, the method using an organic solvent comprises once adjusting pH of an aqueous solution containing prostaglandins to about 3 to convert the prostaglandins into their water-insoluble forms and immediately thereafter extracting them with an organic solvent. Examples of the organic solvents mainly used in the method are ethyl acetate, ether and chloroform. However, such methods using organic solvents require the use of a large amount of organic solvents and are often accompanied by the formation of an emulsion. Moreover, the large amount of organic solvents must be distilled off, which takes a long period of time. Thus, unstable prostaglandins may be decomposed during such a treatment. In addition, pH of the aqueous solution containing the prostaglandins must be controlled to a relatively narrow acidic range in the order of 3 to 3.5 in the method. In this respect, if the pH of the solution is less than 3 due to wrong operations, Prostaglandin E or the like may easily be converted into Prostaglandins A and B. For this reason, the control of pH must be performed with caution and this makes the method complicated. Thus, the methods using organic solvents are disadvantageous from the industrial, economical and operational viewpoints.

On the other hand, examples of the method using resins are those in which non-polar porous synthetic resins such as Amberlite XAD-2 and Amberlite XAD-8 (both available from Rohm & Haas Co., Ltd.). These purification methods utilizing a non-polar porous synthetic resin are excellent in that they do not require the use of a large amount of an organic solvent, but are inferior in the recovery yield and, therefore, are not industrially satisfied methods.

Thus, there have long been desired for the development of methods for purifying prostaglandins which make it possible to eliminate the foregoing problems.

Accordingly, an object of the present invention is to provide a biosynthetic method for preparing prostaglandins in a yield higher than that achieved by the conventional methods.

Another object of the present invention is to provide $PGE_1$ and $PGE_3$ which have not yet been prepared by biosynthetic methods.

A further object of the present invention is to provide an improved method for purifying prostaglandins in which specifically a non-polar porous synthetic resin is used.

According to one aspect of the present invention, there is provided a method for producing prostaglandins which comprises the steps of converting unsaturated fatty acids contained in an alga belonging to genus Gracilaria or genus Hypnea into prostaglandins by the action of an enzyme present in the alga in water or a water-containing solvent so that the prostaglandins are released from the alga into the water or the water-containing solvent and then recovering the prostaglandins from the solvent.

According to another aspect of the present invention, there is provided a method for producing prostaglandins which comprises the steps of adding an unsaturated fatty acid to water or a water-containing solvent, converting the unsaturated fatty acid into prostaglandins by the action of an enzyme present in an alga belonging to genus Gracilaria or genus Hypnea in the solvent and then recovering prostaglandins from the solvent.

According to a further aspect of the present invention, there is provided a method for purifying prostaglandins which comprises the steps of contacting a solution of prostaglandins in water or a water-containing organic solvent with a non-polar porous synthetic resin having a specific surface area of not less than 350 m²/g and a fine pore volume of not less than 0.8 m$\ell$/g to thus adsorb the prostaglandins to the resin and then eluting the adsorbed prostaglandins with an organic solvent or a water-containing organic solvent for elution.

In the light of the fact that the enzymes originated from animal tissues are unstable and expensive, the inventors of this invention have conducted various studies to develop a biosynthetic method for preparing prostaglandins in which the enzyme is from a source other than animal tissues. In the past, it was reported that prostaglandins are present in the algae belonging to genus Gracilaria . Accordingly, the inventors have taken note of, in particular, the content of prostaglandins in the algae and have investigated the algae in detail.

Gracilaria verrucosa (Hudson) Papenfuss was freeze-dried immediately after the collection thereof, then pulverized with a mortar and a pestle to form powder thereof and prostaglandins were intensively extracted with a solvent such as Folch (chloroform : methanol, 2 : 1), acetone and ethanol. After purifying the resulting extracts, they were analyzed by high performance liquid chromatography (hereinafter referred to as "HPLC"), but surprisingly no peak for prostaglandins was observed in either of the extracts (the detection limits of prostaglandins under the conditions herein used were 0.4, 0.1 and 0.1 $\mu$ g per gram of the weight of the wet alga for $PGF_2 \alpha$ , $PGE_2$ and $PGE_1$ , respectively.

In addition, it was also examined on whether the Gracilaria verrucosa (Hudson) Papenfuss living in the sea water releases prostaglandins or not. More specifically, after collecting the Gracilaria verrucosa (Hudson) Papenfuss, 50 g thereof was cultured in 500mℓ of sea water at 15 ° C for 20 days. Subsequently, pH of the sea water was adjusted to 3.5, and extracted with ethyl acetate and the extract was likewise analyzed by HPLC. As a result, it was found that prostaglandin was not present in the sea water at all.

Then the Gracilaria verrucosa (Hudson) Papenfuss was cut into small pieces, water was added thereto and the mixture was stirred. Thereafter, the water phase was separated from the alga, the resulting water phase and the alga were examined on whether prostaglandins were present or not and as a result it was found that not only $PGE_2$ and $PGF_2 \alpha$ but also $PGE_1$ and $PGE_3$ were present in the water phase and that the amount of prostaglandins thus obtained was higher than that produced by the conventional methods. In addition, it was also confirmed that the same result was obtained when algae belonging to genus Hypnea were used.

Moreover, it was also found that if water and arachidonic acid is added to the separated alga and the mixture is stirred, $PGE_2$ and $PGF_2 \alpha$ are obtained and that these operations can be repeatedly performed to further produce prostaglandins. In addition, it was likewise found that $PGE_1$ or $PGE_3$ can be obtained if dihomo-$\gamma$-linolenic acid or eicosapentaenoic acid is employed as a starting fatty acid respectively. In other words, the inventors of this invention have found out that a desired prostaglandin can be prepared by appropriately selecting the kinds of the starting fatty acids and thus have completed the present invention based on such a very important finding.

Incidentally, alga belonging to genus Gracilaria or genus Hypnea do not contain any prostaglandin, but highly unsaturated fatty acids included therein are enzymatically converted into prostaglandins in the presence of water through stimulation such as breakage of tissues or cells of the alga. The foregoing breakage of tissues or cells can be achieved by physical or chemical treatments such as freezing-thawing, cutting into fine pieces, application of ultrasonics, homogenization, pressing and treatments with enzymes.

The algae used in the present invention are not restricted to specific ones so far as they belong to genus Gracilaria or Hypnea , but preferably used are those disclosed in the article of Yamamoto (see H. Yamamoto, Mem. Fac. Fish. , Hokkaido Univ., 1978, 25 (2), p. 97) such as Gracilari a verrucosa (Hudson) Papenfuss; Gracilari a gigas Harvey; and Gracilaria chorda Holmes and preferred examples of those belonging to genus Hypnea are Hypnea charoides Lamouroux; and Hypnea japonica Tanaka.

The algae used in the invention may be those obtained immediately after collection or those which have been stored in a refrigerated or frozen state. In the present invention, alga whose tissues and/or cells are broken are preferably used. The passage "alga whose tissues and/or cells are broken" herein used means and includes, for instance, powdered algae such as those obtained by freeze-drying algae and then pulverizing them to form powder, a crude enzyme or enzymes obtained from these processed materials and those obtained by dispersing the crude enzyme in acetone and collecting the resulting precipitates as well as those immobilized according to the usual enzyme-immobilization method.

In an embodiment of the present invention, the foregoing algae whose tissues and/or cells are broken are added to water or a water-containing solvent, highly unsaturated fatty acids as prostaglandin precursors present in the algae are converted into prostaglandins while making use of an enzyme reaction or reactions (this seems to be a redox reaction) in which the enzyme or enzymes present in the algae are used.

Water usable as a solvent in the invention include, for instance, not only distilled water but also those containing metal ions such as a variety of buffering solutions and sea water. In addition, examples of the water-containing solvents are mixtures comprising water and at least one solvent selected from the group consisting of hydrophilic organic solvents (e.g., methanol, ethanol, n-propanol, isopropanol, n-butanol, acetone and tetrahydrofuran) and hydrophobic organic solvents (e.g., ethyl acetate, ethyl ether, petroleum ether, n-hexane, chloroform, carbon tetrachloride, methylene chloride and benzene).

The enzyme activity may possibly be damaged depending on the kinds of the organic solvent used and, therefore, it should be noted that solvent systems which do not adversely affect the enzyme activity must be employed in the invention. For instance, if an organic solvent which possibly exerts an adverse effect on the enzyme activity depending on the concentration thereof in the water-containing solvent comprising the same such as a lower alcohol is used, the amount of the organic solvent must be limited to a level of not more than 2 parts by weight per one part by weight of water.

The mixing ratio of water to the organic solvent is preferably one part by weight or lower, more preferably not more than 0.5 part by weight per one part by weight of water.

With regard to the ratio of the alga (dry weight) to water in the present invention, the amount of water generally ranges from 1 to 2,000 parts by weight and preferably 5 to 500 parts by weight per one part by weight of the alga. Moreover, the pH value during the enzyme reaction is not limited to a specific value, but it preferably ranges from 3 to 12, more preferably 5 to 11. The temperature of the enzyme reaction may vary over a wide range, but it preferably ranges from 5 to 40° C.

When the enzyme reaction is performed for a desired time according to the present invention, the precursors, i.e., the highly unsaturated fatty acids present in the alga are converted into prostaglandins which are in turn released from the alga into water.

In the method of this invention, the highly unsaturated fatty acid or fatty acids can be intentionally supplemented to the enzyme reaction system. Examples of such fatty acids are those having 16 to 22 carbon atoms and, among these, preferred are arachidonic acid, dihomo-γ-linolenic acid and eicosapentaenoic acid. Salts of these fatty acids may also be employed in the invention. The ratio of the alga to the fatty acid as the starting material may be arbitrarily selected, but the amount of the fatty acid preferably ranges from 0.0001 to 0.1 part by weight per one part by weight of the alga (dry weight). The biosynthetic reaction can proceed even if the reaction system is allowed to stand, but preferably the reaction is performed with stirring. It seems that the stirring makes the reaction system uniform and serves to supplement oxygen in the air to the reaction system. In this respect, a trace amount of oxygen is consumed during the reaction.

When water is used as a reaction solvent, the prostaglandins thus produced are directly extracted from the reaction solution with an organic solvent in the usual manner. Examples of organic solvents usable in such an extraction include ethyl acetate, ethyl ether, petroleum ether, n-hexane, chloroform, carbon tetrachloride, methylene chloride and benzene. On the other hand, when the reaction is carried out in a mixed solvent containing water, the solvent except for water is distilled off under reduced pressure and then the intended products are extracted in the same manner as explained above. The prostaglandins thus obtained can be purified by a method commonly used such as normal phase column chromatography using silica gel or the like and reverse phase column chromatography using ODS or the like.

Alternatively, the prostaglandins thus obtained are more preferably purified by the method for purifying the same according to the present invention. The non-polar porous synthetic resin used in the invention has a specific surface area of not less than 350 m²/g, preferably 450 to 2,000 m²/g and a fine pore volume of not less than 0.8 m ℓ /g, preferably 0.9 to 2.0 mℓ /g. Examples of non-polar porous synthetic resins usable in the invention include Diaion HP 10, 20, 30 and 50, Sepabeads SP 800, 205 and 206 (all of these available from MITSUBISHI CHEMICAL INDUSTRIES LTD.); Amberlite XAD-7 (available from Rohm & Haas Co., Ltd.) and Duolite S-861 (available from Diamond Shamrock Co., Ltd.). Among these, those preferably used in the purification of prostaglandins are Diaion HP 20, Sepabeads SP 205, Sepabeads SP 206 and Duolite S-861. The Diaion HP type resins, Sepabeads SP 800 and Duolite S-861 are non-polar porous synthetic resins of styrene/divinylbenzene type. In addition, Sepabeads SP 205 and Sepabeads SP 206 are those obtained by introducing hydrophobic substituents in matrices of non-polar porous synthetic resins of styrene/divinylbenzene type. Moreover, Amberlite XAD-7 is an acrylate type non-polar porous synthetic resin.

The purification method of the present invention can be performed by passing a solution of prostaglandins in water or a water- containing organic solvent through a column packed with the foregoing non-polar porous synthetic resin to thus adsorb the prostaglandins to the resin. The aqueous solution containing prostaglandins may comprise a salt such as sodium chloride for the purpose of promoting the adsorption of prostaglandins.

Alternatively, it is also possible to use an aqueous solution containing an organic solvent in an amount which does not adversely affect the adsorption of prostaglandins to the porous synthetic resin. Examples of such water-containing organic solvents are mixtures of water with methanol, ethanol, isopropanol and/or acetone. For instance, if it comprises water and methanol, the amount of methanol is in general not more than 0.5 part by weight, preferably not more than 0.45 part by weight per one part by weight of water.

After adsorbing the prostaglandins, the non-polar porous synthetic resin is washed with a small amount of water or a water-containing organic solvent in an amount which does not cause the elution of the prostaglandins. Then the adsorbed prostaglandins are eluted with an organic solvent or a water-containing organic solvent for elution to thus give the prostaglandins in a high concentration.

Examples of the organic solvents for eluting the prostaglandins are methanol, ethanol, isopropanol and acetone. Examples of the water-containing organic solvents for elution are the foregoing organic solvents to which a small amount of water is added. For instance, if it comprises methanol and water, the amount of water is generally not more than 0.5 part by weight, preferably 0.45 part by weight per one part by weight of methanol.

In the present invention, the non-polar porous synthetic resin may be regenerated with the use of an organic solvent, an acid or an alkali to reuse the same. Examples of the solvents used in the regeneration are methanol, ethanol, isopropanol, benzene, carbon tetrachloride and acetone, those of alkalis include sodium hydroxide and ammonia and the acid may be hypochlorous acid.

According to the method of this invention, prostaglandins can be obtained, in high yield, from highly unsaturated fatty acids using the algae belonging to genus Gracilaria or genus Hypnea . Unlike the conventional biosynthetic methods which are limited in the yield since internal organs of animals are employed as a source for the enzymes, the method of this invention makes it possible to achieve mass production of prostaglandins since the algae belonging to genus Gracilaria or genus Hypnea as a source for the enzyme are easily available. In addition, the conventional extraction methods cannot provide a specific kind of prostaglandin selectively, but the method of the present invention ensures the production of a desired prostaglandin in high yield if the kind of the starting fatty acid is appropriately selected. The prostaglandins thus produced can widely be used in the field of medicament as a medicine for treating chronic arterial obstruction, an aparturifacient in the field of obsterics and gynecology and a promotor of intestinal movement after abdominal operations.

In the method for purifying prostaglandins according to the present invention, a specific non-polar porous synthetic resin can selectively and specifically adsorb prostaglandins in a solution method makes it possible to recover the prostaglandins in high yield. In other words, the method is an industrially effective one for purifying prostaglandins.

The present invention will hereinafter be explained in more detail with reference to the following non-limitative working Examples. Moreover, the effects practically achieved by the present invention will also be discussed in detail in comparison with Comparative Examples.

Example 1

Gracilaria verrucosa (Hudson) Papenfuss collected at Miura Peninsula in Kanagawa Prefecture was cut into fine pieces with a food processor (available from Matsushita Electric Industrial Co., Ltd. under the trade name of National Speed Cutter MK-K5). To 5 g (dry weight = 785 mg) of the fine pieces, there was added 50mℓ of distilled water and the mixture was stirred at 5° C overnight. Then pH of the reaction solution was adjusted to 3 to 4 with the addition of hydrochloric acid and the solution was extracted with an equivalent volume of ethyl acetate. The resulting ethyl acetate phase was washed with water and the solvent was distilled off under reduced pressure to obtain residue. The residue was pretreated with an SEP-PAK $C_{18}$ cartridge. More specifically, the prostaglandin fraction was suspended and/or dissolved in a 15% ethanol aqueous solution, charged in the SEP-PAX $C_{18}$ cartridge and eluted in order with 10mℓ of a 15% ethanol aqueous solution, 10 mℓ of hexane and 10mℓ of ethyl acetate. The ethyl acetate fraction is recovered as a prostaglandin fraction. Then this fraction was subjected to HPLC. The HPLC was carried out using ODS-AQ (6 X 150 mm; available from YMC Company) as a column; 0.017 M phosphoric acid/acetonitrile (65:35) as a mobile phase, the detection of prostaglandins was performed using UV absorption spectroscopy at 196 nm and the HPLC was performed at a flow rate of 1.5 mℓ /min. As a result, peaks which were likely to indicate the presence of $PGE_3$ , $PGF_2 \alpha$ , $PGE_2$ and $PGE_1$ were observed at 8.6, 9.8, 12.2 and 13.4 min, respectively. These peaks were all in agreement with the effluence positions of every standard substances, respectively. Moreover, the fractions showing these peaks were collected and analyzed by the bioassay of Magnus, radioimmunoassay, gas chromatography (hereunder referred to as "GC"), gas chromatography/mass spectroscopy analysis (hereunder referred to as "GC/MS") and nuclear magnetic resonance spectroscopic analysis (hereunder referred to as "NMR") to confirm the structures thereof.

The prostaglandins thus obtained from Gracilaria verrucosa (Hudson) Papenfuss were compared with the standard substances using GC/MS analysis and the characteristic peaks thereof observed were summarized in the following Table I. The prostaglandins were converted into methyl esters at the carboxyl group, trimethylsilyl ethers at the hydroxyl group and methyl oximes at the keto group and the resulting derivatives of the prostaglandins were analyzed by chemical ionization technique (ammonia; $1.5 \times 10^{-5}$ mmHg). In Table I, the terms "Peak I" and "Peak II" represent syn-anti isomers formed during converting the keto group existing at C-9 position into methyl oximes.

Table I

| Comparison of GC/MS Data Between The Products of the Invention and Standard Substances | |
|---|---|
| Sample | m/z |
| PGE$_1$ (Standard) | Peak I 542 (MH$^+$), 452, 296, 279, 191, 90 |
| PGE$_1$ (Standard) | Peak II 542 (MH$^+$), 452, 191, 90 |
| PGE$_1$ (Example) | Peak I 542 (MH$^+$), 452, 296, 279, 191, 90 |
| PGE$_1$ (Example) | Peak II 542 (MH$^+$), 452, 191, 90 |
| PGE$_2$ (Standard) | Peak I 540 (MH$^+$), 450, 277, 191, 90 |
| PGE$_2$ (Standard) | Peak II 540 (MH$^+$), 450, 160, 148, 90 |
| PGE$_2$ (Example) | Peak I 540 (MH$^+$), 450, 277, 191, 90 |
| PGE$_2$ (Example) | Peak II 540 (MH$^+$), 450, 160, 148, 90 |
| PGE$_3$ (Standard) | Peak I 538 (MH$^+$), 448, 396, 275, 191, 90 |
| PGE$_3$ (Standard) | Peak II 538 (MH$^+$), 448, 396, 191, 90 |
| PGE$_3$ (Example) | Peak I 538 (MH$^+$), 448, 396, 275, 191, 90 |
| PGE$_3$ (Example) | Peak II 538 (MH$^+$), 448, 396, 191, 90 |
| PGF$_2$ $\alpha$ (Standard) | 585 (MH$^+$), 495, 315, 90 |
| PGF$_2$ $\alpha$ (Example) | 585 (MH$^+$), 495, 315, 90 |

The prostaglandins thus obtained from Gracilaria verrucosa (Hudson) Papenfuss were compared with the standard substances using $^1$H-NMR spectroscopic analysis. The results thus observed are summarized in the following Table II. In the NMR measurement, there were used, as a solvent, CDCl$_3$ :CD$_3$OD (9:1) for PGE$_1$ ; CDCl$_3$ for PGE$_2$ , PGE$_3$ and PGF$_2$ $\alpha$ , respectively and TMS was used as an internal standard.

Table II

| Comparison of $^1$H-NMR Data Between The Products of the Invention and Standard Substances (ppm) | | | | |
|---|---|---|---|---|
| Prostaglandin | Standard Substance | | Product of Example | |
| PGE$_1$ | 5.62 | 1H, dd | 5.62 | 1H, dd |
| | 5.51 | 1H, dd | 5.52 | 1H, dd |
| | 4.07~3.98 | 2H, m | 4.07~3.97 | 2H, m |
| | 2.72 | 1H, dd | 2.72 | 1H, dd |
| | 0.89 | 3H, t | 0.89 | 3H, t |
| PGE$_2$ | 5.67 | 1H, dd | 5.64 | 1H, dd |
| | 5.57 | 1H, dd | 5.55 | 1H, dd |
| | 5.39 | 2H, m | 5.38 | 2H, m |
| | 4.15~4.02 | 2H, m | 4.12~4.01 | 2H, m |
| | 2.75 | 1H, dd | 2.74 | 1H, dd |
| | 0.89 | 3H, t | 0.89 | 3H, t |
| PGE$_3$ | 5.78~5.59 | 3H, m | 5.75~5.56 | 3H, m |
| | 5.53~5.33 | 3H, m | 5.47~5.31 | 3H, m |
| | 4.30~4.25 | 1H, m | 4.24~4.20 | 1H, m |
| | 4.14~4.07 | 1H, m | 4.11~4.04 | 1H, m |
| | 2.75 | 1H, dd | 2.75 | 1H, dd |
| | 0.98 | 3H, t | 0.98 | 3H, t |
| PGF$_2$ $\alpha$ | 5.60~5.32 | 4H, m | 5.61~5.33 | 4H, m |
| | 4.17~3.91 | 3H, m | 4.17~3.90 | 3H, m |
| | 0.89 | 3H, t | 0.89 | 3H, t |
| dd: doublet doublet; m: multiplet; t : triplet. | | | | |

The prostaglandins thus obtained from Gracilaria verrucosa (Hudson) Papenfuss were compared with the standard substances using $^{13}$C-NMR Spectroscopic analysis. The results thus observed are summarized in the following Table III. In the NMR measurement, there were used, as a solvent, $CDCl_3$ :$CD_3OD$ (9:1) for PGE$_1$ ; $CDCl_3$ for PGE$_2$ and PGF$_2$ $\alpha$ , respectively and TMS was used as an internal standard.

Table III

| Comparison $^{13}$C-NMR Data Between The Products of the Invention and Standard Substances (ppm) | | | | | | |
|---|---|---|---|---|---|---|
| | PGE$_1$ | | PGE$_2$ | | PGF$_2$ $\alpha$ | |
| | Stan. | Inv. | Stan. | Inv. | Stan. | Inv. |
| C(1) | 176.6 | 176.7 | 177.7 | 177.6 | 177.5 | 177.3 |
| C(2) | 34.1 | 34.2 | 33.1 | 33.2 | 33.1 | 32.9 |
| C(3) | 24.9 | 25.0 | 24.4 | 24.5 | 24.7 | 24.5 |
| C(4) | 29.4 | 29.5 | 26.3 | 26.4 | 26.5 | 26.3 |
| C(5) | 28.9 | 29.0 | 130.8 | 130.9 | 132.9 | 132.7 |
| C(6) | 26.6 | 26.7 | 126.7 | 126.7 | 129.4 | 129.2 |
| C(7) | 27.7 | 27.8 | 25.2 | 25.2 | 25.4 | 25.3 |
| C(8) | 54.8 | 54.8 | 54.5 | 54.5 | 50.4 | 50.2 |
| C(9) | 216.1 | 216.4 | 214.4 | 214.7 | 72.6 | 72.5 |
| C(10) | 46.1 | 46.2 | 46.2 | 46.2 | 42.9 | 42.7 |
| C(11) | 71.6 | 71.7 | 72.2 | 72.1 | 77.8 | 77.6 |
| C(12) | 54.6 | 54.6 | 53.5 | 53.5 | 55.5 | 55.3 |
| C(13) | 132.0 | 132.0 | 131.2 | 131.5 | 129.8 | 129.6 |
| C(14) | 136.9 | 136.9 | 136.5 | 136.6 | 135.2 | 135.0 |
| C(15) | 73.1 | 73.1 | 73.2 | 73.2 | 73.4 | 73.2 |
| C(16) | 37.0 | 37.0 | 36.9 | 36.9 | 37.1 | 36.9 |
| C(17) | 25.3 | 25.3 | 25.2 | 25.4 | 25.4 | 25.2 |
| C(18) | 31.8 | 31.9 | 31.7 | 31.7 | 31.9 | 31.7 |
| C(19) | 22.7 | 22.8 | 22.6 | 22.6 | 22.8 | 22.6 |
| C(20) | 14.0 | 14.1 | 14.0 | 14.1 | 14.2 | 14.1 |

Comparative Example 1

Prostaglandins were prepared according to the method disclosed in J.P. KOKOKU No. Hei 1-14910. More specifically, the same Gracilaria verrucosa (Hudson) Papenfuss as that used in Example 1 was frozen at -20 °C, 5 g (dry weight = 1080 mg) thereof was cut into fine pieces and immersed in 20mℓ of ethanol overnight. The solution obtained through the immersion was concentrated under reduced pressure, water was added to the concentrate, pH of the mixture was adjusted to 3 to 4 by the addition of hydrochloric acid and then the prostaglandins were extracted and quantitatively determined as in Example 1.

The amounts ( $\mu$ g/g wet weight) of prostaglandins produced in Example 1 and Comparative Example 1 are listed in the following Table IV, respectively.

Table IV

| Kind of Prostaglandin | Example 1 | Comp. Example 1 |
|---|---|---|
| PGE$_1$ | 32.1 | 0 |
| PGE$_2$ | 413.1 | 2.0 |
| PGE$_3$ | 1.4 | 0 |
| PGF$_2$ $\alpha$ | 2.3 | 0 |

In the method of Example 1, the fats present in Gracilaria verrucosa (Hudson) Papenfuss were converted into methyl esters of the fatty acids by an ester exchange reaction in which sodium methoxide was used and the usual manner using diazomethane and were analyzed by GC. As a result, it was confirmed that after the enzyme reaction, the unsaturated fatty acids were consumed as the substrate for the enzyme reaction and hence decreased. The results observed are summarized in Table V given below. The numerical values in Table V are expressed in μ g/g wet weight.

Table V

| Fatty Acid | Methyl Dihomo- γ linolinate | Methyl Arachidonate | Methyl Eicosapentaenate |
|---|---|---|---|
| Prior to Enzyme Reaction After Enzyme Reaction | 72.3 13.7 | 882.7 308.0 | 50.8 21.5 |

Example 2

Gracilaria verrucosa (Hudson) Papenfuss collected at Miura Peninsula in Kanagawa Prefecture was frozen at -20 ° C. 5 g (dry weight = 970 mg) was cut into fine pieces. To the fine pieces, there was added 20mℓ of distilled water or a 25% or 50% aqueous ethanol solution or ethanol to perform immersion overnight. After the reaction, prostaglandins obtained were extracted and quantitatively determined as in Example 1. The amounts ( μ g/g wet weight) of prostaglandins produced are listed in the following Table VI.

Table VI

| Kind of Prostaglandin | Distilled Water | 25% Ethanol Solution | 50% Ethanol Solution | Ethanol* |
|---|---|---|---|---|
| PGE$_1$ | 8.4 | 9.0 | 8.2 | 0 |
| PGE$_2$ | 134.7 | 106.7 | 86.1 | 2.0 |
| PGE$_3$ | 0.7 | 0.6 | 0.9 | 0 |
| PGF$_2$ α | 5.0 | 4.6 | 5.0 | 0 |

* Comperative example

Example 3

Gracilaria verrucosa (Hudson) Papenfuss collected at Miura Peninsula in Kanagawa Prefecture was freeze-dried and powdered with a mortar and a pestle. To 500 mg (wet weight = 3.5 g) of the powdered alga, 35 m ℓ of distilled water was added and the mixture was stirred at 5° C overnight. After the reaction, prostaglandins obtained were extracted and quantitatively determined as in Example 1. The amounts (μ g/g wet weight) of prostaglandins produced are listed in the following Table VII.

Table VII

| Kind of Prostaglandin | Amount Produced |
|---|---|
| PGE$_1$ | 22.7 |
| PGE$_2$ | 217.7 |
| PGE$_3$ | 1.1 |
| PGF$_2$ α | 1.7 |

10

Example 4

10 g (dry weight = 1140 mg) of Hypnea charoides Lamouroux collected at Onagawa-Cho in Miyagi Prefecture was cut into fine pieces in the same manner used in Example 1, 50 mℓ of distilled water was added to the fine pieces and the mixture was stirred at 5 ° C overnight. After the reaction, prostaglandins obtained were extracted and quantitatively determined as in Example 1. The amounts ($\mu$ g/g wet weight) of prostaglandins produced are listed in the following Table VIII.

Table VIII

| Kind of Prostaglandin | Amount Produced |
|---|---|
| PGE$_1$ | 12.6 |
| PGE$_2$ | 132.5 |
| PGE$_3$ | 2.6 |
| PGF$_2$ $\alpha$ | 4.9 |

Example 5

Hypnea charoides Lamouroux collected at Onagawa-Cho in Miyagi Prefecture was freeze-dried, 20 m ℓ of distilled water was added to 100 mg (wet weight = 877 mg) of the freeze-dried alga and the mixture was stirred at 5 ° C overnight. After the reaction, prostaglandins obtained were extracted and quantitatively determined as in Example 1. The amounts ($\mu$ g/g wet weight) of prostaglandins produced are listed in the following Table IX.

Table IX

| Kind of Prostaglandin | Amount Produced |
|---|---|
| PGE$_1$ | 8.9 |
| PGE$_2$ | 213.2 |
| PGE$_3$ | 1.1 |
| PGF$_2$ $\alpha$ | 4.2 |

Example 6

Gracilaria verrucosa (Hudson) Papenfuss collected at Miura Peninsula in Kanagawa Prefecture was cut into fine pieces, 50 mℓ of distilled water was added to 5 g (dry weight = 735 mg) of the fine pieces and the reaction was performed at room temperature for one hour with stirring so that the fatty acids present in the Gracilaria verrucosa (Hudson) Papenfuss were consumed as much as possible. Thereafter, the reaction solution was subjected to filtration to separate the alga fr22 the filtrate. 50 m ℓ of distilled water was added to the resulting alga, followed by addition of 100$\mu$ ℓ of an ethanol solution containing 10 mg of dihomo- $\gamma$ - linolenic acid and stirring at room temperature for one hour. After the reaction, the alga was separated from the filtrate in the same manner as used above and the amounts of prostaglandins present in the filtrate were determined as in Example 1. The amounts of prostaglandins produced ( $\mu$ g/g wet weight) are listed in the following Table X together with the results of the blank test in which dihomo-$\gamma$-linolenic acid was not used.

Table X

| Kind of Prostaglandin | Example | Blank |
|---|---|---|
| PGE$_1$ | 31.4 | 0.4 |
| PGE$_2$ | 5.2 | 3.5 |
| PGE$_3$ | 0 | 0 |
| PGF$_2$ $\alpha$ | 0 | 0 |

Example 7

The same procedures used in Example 6 were carried out except that eicosapentaenoic acid was substituted for the dihomo-$\gamma$-linolenic acid used in Example 6 to produce prostaglandins. The amounts of prostaglandins produced ($\mu$ g/g wet weight) are listed in the following Table XI.

Table XI

| Kind of Prostaglandin | Example | Blank |
|---|---|---|
| PGE$_1$ | 0 | 0 |
| PGE$_2$ | 7.4 | 3.5 |
| PGE$_3$ | 25.7 | 0 |
| PGF$_2$ $\alpha$ | 0 | 0 |

Example 8

The same procedures used in Example 6 were carried out except that arachidonic acid was substituted for the dihomo-$\gamma$-linolenic acid used in Example 6 and that these procedures were repeated twice to produce prostaglandins. The amounts of prostaglandins produced ($\mu$ g/g wet weight) are listed in the following Table XII.

Table XII

| Kind of Prostaglandin | Example | | Blank | |
|---|---|---|---|---|
| | 1st Reac. | 2nd Reac. | 1st Reac. | 2nd Reac. |
| PGE$_1$ | 0 | 0 | 0 | 0 |
| PGE$_2$ | 132.3 | 42.0 | 3.6 | 1.9 |
| PGE$_3$ | 0 | 0 | 0 | 0 |
| PGF$_2$ $\alpha$ | 2.7 | 1.2 | 0.2 | 0.1 |

Example 9

1 kg (dry weight = 146 g) of Gracilaria verrucosa (Hudson) Papenfuss which had been collected at Miura Peninsula in Kanagawa Prefecture and had been frozen at -20 ° C was immersed in 2 ℓ of distilled water at room temperature overnight to perform a reaction. Thereafter, the alga was separated from the reaction solution and additionally 750mℓ of distilled water was added to the separated alga to perform further reaction. The same operations were repeated 5 times. Prostaglandins obtained were extracted from the resulting combined reaction solution (about 6.0 ℓ in all) and quantitatively determined ($\mu$ g/g of wet alga) in the same manner used in Example 1. The results obtained are summarized in the following Table XIII.

Table XIII

| Kind of Prostaglandin | Amount Produced |
|---|---|
| PGE$_1$ | 8.6 |
| PGE$_2$ | 222.0 |
| PGE$_3$ | 3.0 |
| PGF$_2$ $\alpha$ | 5.4 |

Example 10

The resins used in the following Examples and Comparative Examples are detailed in Table XIV given below:

Table XIV

| Resin (Trade Name) | Sp. Area ($m^2$/g) | Pore Volume (m ℓ/g) | Resin Matrix |
|---|---|---|---|
| A (Diaion HP 20) | 511 | 1.18 | styrene-divinylbenzene |
| B (Sepabeads SP 205) | 507 | 1.04 | styrene-divinylbenzene (substituents are introduced) |
| C (Sepabeads SP 206) | 556 | 0.94 | ditto |
| D (Sepabeads SP 800) | 819 | 0.93 | styrene-divinylbenzene |
| E (Duolite S-861) | 500~550 | 0.8~0.9 | ditto |
| F (Amberlite XAD-7) | 450 | 1.14 | acrylate type |
| X (Amberlite XAD-2) | 300 | 0.65 | styrene-divinylbenzene |
| Y (Amberlite XAD-8) | 140 | 0.79 | acrylate type |

According to the similar procedures in Example 9, 260 ℓ of an aqueous solution containing prostaglandins was obtained from 60 kg of Gracilaria verrucosa (Hudson) Papenfuss collected at Miura Peninsula in Kanagawa Prefecture. The amounts of the resulting prostaglandins were quantitatively determined as in Example 1. As a result, 401 mg of PGE$_1$ , 11,794 mg of PGE$_2$ , 198 mg of PGE$_3$ and 327 mg of PGF$_2$ $\alpha$ were obtained.

The aqueous solution was passed through a column packed with 1.5 ℓ of the resin A at a flow rate equal to a space velocity (SV: this means the ratio of the volume of the aqueous solution passed therethrough per volume of the resin in an hour) of 10 to adsorb the prostaglandins. Subsequently, the column was washed with 7.5 ℓ of water and then the adsorbed prostaglandins were eluted with 4.5 ℓ of methanol to give fractions containing the corresponging prostaglandins. The same aqueous solution was likewise treated with columns each packed with the resin B and the resin X as a comparative example to thus give every fractions containing the corresponding prostaglandins. In addition, the same procedures were likewise repeated using 6 ℓ of the resin X. The amounts of the prostaglandins in each fraction obtained were likewise quantitatively determined by HPLC and the recovery yields were determined in each case while the amounts of the prostaglandins in the aqueous solution prior to the treatment with the column packed with the adsorption resin was defined to be 100 %. The results thus obtained are summarized in the following Table XV.

Table XV

| Recovery Yield Achieved by Various Porous Synthetic Resins | | | | |
|---|---|---|---|---|
| Resin | Prostaglandin (%) | | | |
| | $PGE_1$ | $PGE_2$ | $PGE_3$ | $PGF_2\alpha$ |
| A | 100 | 100 | 92 | 100 |
| B | 76 | 87 | 100 | 96 |
| X (Comp. Ex.) | 38 | 42 | 19 | 40 |
| X (Comp. Ex.: 6 ℓ) | 41 | 46 | 23 | 38 |

As shown in Table XV, the method of the present invention in which specific non-polar porous synthetic resins are employed can achieve high recovery yield, on the contrary when the resin X is employed (Comparative Example), the recovery yield is substantially lowered and the recovery yield is not enhanced even if the resin X is employed in an amount of 6ℓ in the Comparative Example.

Example 11

The same procedures used in Example 10 were carried out except for using 5 g of the Gracilaria verrucosa (Hudson) Papenfuss to give 24 mℓ of an aqueous solution containing prostaglandins. The amounts of the resulting prostaglandins were quantitatively determined by HPLC as in Example 1. As a result, 48μ g of $PGE_1$ , 1,014 μ g of $PGE_2$, 10 μg of $PGE_3$ and 25μ g of $PGF_2\alpha$ were obtained.

The aqueous solution was passed through a column packed with 8 m ℓ of a resin A at an SV of 40 to adsorb the prostaglandins. Subsequently, the column was washed with 100 m ℓ of distilled water and then the adsorbed prostaglandins were eluted with 40 mℓ of methanol to give fractions containing the corresponging prostaglandins. The same aqueous solution was likewise treated with columns each packed with the resins C, D, E and F and the resin Y as a comparative example to thus give every fractions containing the corresponding prostaglandins. The amounts of the prostaglandins in each fraction obtained were likewise quantitatively determined by HPLC as in Example 1 and the recovery yields were determined in each case while the amounts of the prostaglandins in the aqueous solution prior to the treatment with the column packed with the adsorption resin were defined to be 100 %. The results thus obtained are summarized in the following Table XVI.

Table XVI

| Recovery Yield Achieved by Various Porous Synthetic Resins | | | | |
|---|---|---|---|---|
| Resin | Prostaglandin (%) | | | |
| | $PGE_1$ | $PGE_2$ | $PGE_3$ | $PGF_2\alpha$ |
| A | 98 | 100 | 97 | 100 |
| C | 100 | 89 | 92 | 81 |
| D | 79 | 74 | 76 | 65 |
| E | 100 | 98 | 81 | 87 |
| F | 70 | 71 | 73 | 59 |
| Y (Comp. Ex.) | 43 | 45 | 29 | 35 |

## Claims

1. A method for producing prostaglandins comprising the steps of converting unsaturated fatty acids contained in alga belonging to genus Graciaria or genus Hypnea into prostaglandins by the action of an enzyme present in the alga in water or a water-containing solvent so that the prostaglandins are released from the alga into the water or the water-containing solvent and then recovering the prostaglandins from the solvent.

2. The method of claim 1 wherein the alga belonging to genus Gracilaria or genus Hypnea is Gracilaria verrucos a (Hudson) Papenfuss, Gracilaria gigas Harvey, Gracilaria chorda Holmes, Hypnea charoides Lamouroux or Hypnea japonica Tanaka.

3. The method of claim 1 or 2 wherein the alga is one obtained by breakage of tissues or cells thereof by a physical or chemical operation.

4. The method of claim 3 wherein the physical or chemical operation is a freeze-drying and pulverizing operation, a freezing-thawing operation, a cutting operation into fine pieces, application of ultrasonics, homogenization, pressing or a treatment with an enzyme.

5. The method of claim 3 or 4 wherein the alga whose tissues and/or cells are broken are powdered algae, a crude enzyme or enzymes obtained from said powdered algae, a crude enzyme or enzymes obtained by dispersing said crude enzymes in acetone and collecting the resulting precipitates or enzymes immobilized according to an enzyme-immobilization method.

6. The method of any one of claims 1 to 5 wherein the ratio of the alga on dry basis to water ranges from 1 to 2,000 parts by weight per one part by weight of the alga, the pH value during the enzyme reaction ranges from 3 to 12 and the temperature of the enzyme reaction ranges from 5 to 40 $^\circ$ C.

7. The method of any one of claims 1 to 6 wherein the prostaglandins are prostaglandin $E_1$ , prostaglandin $E_2$ , prostaglandin $E_3$ or prostaglandin $F_2$ $\alpha$.

8. A method for producing prostaglandins comprising the steps of adding an unsaturated fatty acid to water or a water-containing solvent, converting the unsaturated fatty acid into prostaglandins in the solvent by the action of an enzyme present in an alga belonging to genus Gracilaria or genus Hypnea and then recovering prostaglandins from the solvent.

9. The method of claim 8 wherein the highly unsaturated fatty acid contains from 16 to 22 carbon atoms.

10. The method of claim 8 or 9 wherein the highly unsaturated fatty acid is arachidonic acid, dihomo-$\gamma$ - linolenic acid, eicosapentaenoic acid or salts of these fatty acids.

11. The method of any one of claims 8 to 1,0 wherein the amount of the fatty acid as the starting material ranges from 0.0001 to 0.1 part by weight per one part by weight of the alga body (dry weight).

12. The method of any one of claims 1 to 11 wherein water is distilled water and the water-containing solvent is a mixture comprising water and at least one solvent which is methanol, ethanol, n-propanol, isopropanol, n-butanol, acetone, tetrahydrofuran, ethyl acetate, ethyl ether, petroleum ether, n-hexane, chloroform, carbon tetrachloride, methylene chloride or benzene.

13. The method of any one of claims 8 to 12 wherein the addition of the fatty acid, the conversion thereof into prostaglandins and the recovery of the resulting prostaglandins are repeated.

14. The method of any one of claims 1 to 13 wherein the prostaglandins recovered are purified by contacting a solution of the prostaglandins in water or a water-containing organic solvent with a non-polar porous synthetic resin having a specific surface area of not less than 350 m$^2$/g and a fine pore volume of not less than 0.8 m$\ell$ /g to thus adsorb the prostaglandins to the resin and then eluting the adsorbed prostaglandins with an organic solvent or a water-containing organic solvent for elution.

15. A method for purifying prostaglandins comprising the steps of contacting a solution of prostaglandins in water or a water-containing organic solvent with a non-polar porous synthetic resin having a specific surface area of not less than 350 m$^2$/g and a fine pore volume of not less than 0.8 m $\ell$ /g to thus adsorb the prostaglandins to the resin and then eluting the adsorbed prostaglandins with an organic solvent or a water-containing organic solvent for elution.

16. The method of claim 15 wherein the non-polar porous synthetic resin is one having a specific surface area ranging from 450 to 2,000 m$^2$/g and a fine pore volume ranging from 0.9 to 2.0 m $\ell$ /g.

17. The method of claim 15 or 16 wherein the water-containing organic solvent comprises water and an organic solvent which is methanol, ethanol, isopropanol or acetone in an amount of not more than 0.5 part by weight per one part by weight of water and the water-containing organic solvent for elution comprises water and an organic solvent which is methanol, ethanol, isopropanol or acetone, in a ratio of not more than 0.5 part by weight of water per one part by weight of the organic solvent.